Europäisches Patentamt

European Patent Office

Office européen des brevets

(11) Veröffentlichungsnummer: **0 467 137 A2**

## (12) EUROPÄISCHE PATENTANMELDUNG

(21) Anmeldenummer: **91110822.3**

(51) Int. Cl.⁵: **G05D 25/02**

(22) Anmeldetag: **29.06.91**

(30) Priorität: **14.07.90 DE 4022465**

(43) Veröffentlichungstag der Anmeldung:
**22.01.92 Patentblatt 92/04**

(84) Benannte Vertragsstaaten:
**DE FR GB IT**

(71) Anmelder: **DORNIER MEDIZINTECHNIK GMBH**
**Postfach 1128**
**W-8000 München(DE)**

(72) Erfinder: **Werner, Harald, Dipl.-Ing.**
**Liesel-Beckmannstrasse 5**
**W-8000 München 70(DE)**
Erfinder: **Ibler, Robert, Dipl.-Ing.**
**Labertalstrasse 14**
**W-8411 Alling(DE)**
Erfinder: **Lumma, Detlev, Dipl.-Phys.**
**Hörwegstrasse 31**
**W-8034 Germering(DE)**
Erfinder: **Buck, Roland**
**Südendstrasse 69**
**W-8034 Germering(DE)**
Erfinder: **Obbarius, Hans-Ulrich, Dr.-Phys.**
**Preehlerweg 19a**
**W-8031 Gilching(DE)**

(74) Vertreter: **Kasseckert, Rainer**
**DORNIER GMBH Kleeweg 3**
**W-7990 Friedrichshafen 1(DE)**

(54) **Laserenergiemessung und -regelung.**

(57) Es wird eine Vorrichtung zum Messen und zum Regeln der am distalen Ende einer Lichtleitfaser (GF) emittierten Laserenergie eines insbesondere medizinischen Lasergeräts (Laserlithotripter) vorgeschlagen. Sie umfasst eine Kalibriereinheit (KE), eine Monitoreinheit (ME) und eine Meß- und Regeleinheit (MR). In der Kalibrierroutine wird die von der Kalibriereinheit (KE) gemessene distale Energie mit der aus dem Laser (L) austretenden, von der Monitoreinheit (ME) gemessenen Energie verglichen. Während der Behandlung steuert die Meß- und Regeleinheit (MR) den Laser (L) anhand der vorher ermittelten Energiewerte und der aktuellen von der Monitoreinheit (ME) ermittelten Werte.

Fig.1

Die Erfindung betrifft eine Vorrichtung nach dem Oberbegriff des Anspruchs 1 oder des Anspruchs 4.

Bei bestimmten Lasern, z.B. gepulsten Hochleistungslasern für die Medizintechnik, wie für die Laserlithotripsie, ist es mit Hilfe einer Glasfaser möglich, die Laserenergie auch an schwer zugänglichen Stellen wie z.B. Körperhöhlen einzusetzen. Dabei ist es wünschenswert, die Energie am distalen Faserende zu messen oder am Laser einstellen zu können, um z.B. zu hohe Energien und damit eine Gefährdung des Patienten zu vermeiden.

Das Problem besteht nun erstens darin, daß man weder am Behandlungsort noch zur Behandlungszeit die distal abgegebene Energie messen kann. Zweitens müßte eine in Frage kommende Meßvorrichtung sowohl sehr hohe Leistungsdichten (wie sie in der Laserlithotripsie benötigt werden) verarbeiten und auf der anderen Seite eine Ortsunabhängigkeit aufweisen, da die Austrittsrichtung des Laserstrahls aus der Lichtleitfaser differieren kann.

Aufgabe der Erfindung ist es, eine Messvorrichtung und eine Regelung vorzuschlagen, mit der die Energie am Faserende auf einen gewünschten Wert eingestellt und gehalten werden kann, wodurch der Patient sicher vor zu hohen Energien geschützt wird, wodurch reproduzierbare Ergebnisse erzielbar sind.

Diese Aufgabe wird erfindungsgemäß gelöst von einer Vorrichtung mit den Merkmalen des Anspruchs 1 oder des Anspruchs 4. Ausführungen der Erfindung ist Gegenstände von Unteransprüchen.

Erfindungsgemäß kann auf eine unmittelbare Messung während der Behandlung verzichtet werden, da in einer vorher ablaufenden Kalibrierroutine die für die Dauer einer Behandlung gewünschte distale Energie in einem engen Toleranzbereich gewährleistet werden kann. Dies wird mit der erfindungsgemässen Vorrichtung realisiert.

Die erfindungsgemäße Regelvorrichtung umfasst hauptsächlich zwei Baugruppen, die sogenannte Monitoreinheit und die Kalibriereinheit. Die Monitoreinheit mißt während des Kalibrierens und während des eigentlichen Betriebs das vom Laser emittierte Licht. Sie befindet sich z.B. am Laser (Laserkopf) hinter dem hochreflektierenden Spiegel oder im Strahlengang hinter dem Auskoppelspiegel und mißt mit Hilfe einer Photodiode, die so gewählt ist, daß sie die Laserpulse zeitlich erfassen kann, sowie einer nachfolgenden Auswerteschaltung die vom Resonator abgegebene Laserenergie. Die Energiemessung hinter dem Laser hat den Vorteil, daß die Nutzenergie nicht gestört wird. Die geringe Resttransmission des hochreflektierenden Spiegels dämpft zudem die hohe Energie auf eine so niedrige Schwelle, daß die messende Photodiode nicht zerstört wird.

Die Kalibriereinheit enthält ebenfalls eine Photodiode und eine nachgeschaltete Auswerteschaltung. Das Problem der Zerstörung der Meßeinrichtung durch die hohe Laserleistung kann durch Abschwächer oder durch geeignete Filter gelöst werden. In einer bevorzugten Ausführung wird eine Ulbricht-Kugel verwendet. Diese Hohlkugel hat zwei Öffnungen, die erste ist die Eintrittsöffnung für die Laserstrahlung, die zweite ist die Austrittsöffnung, hinter der die Photodiode sitzt. Die Kugel ist innen mit einer weissen, diffusen Beschichtung ausgekleidet, die die einfallende Laserstrahlung durch diffuse Reflexion gleichmäßig über die Kugelinnenfläche verteilt und somit die hohe Leistungsdichte am Faserende herabsetzt. Dadurch wird es möglich, mit einer Photodiode die Relativmessung der distalen Laserenergie durchzuführen. Die räumliche Verteilung des Laserstrahls beeinflusst das Meßergebnis nicht. Die in Anspruch 4 angegebene Meßvorrichtung ist besonders für stabil arbeitende Laser geeignet, bei denen die Ausgangsleistung über viele Schuß hinweg zuverlässig mit der Eingangs- oder Pumpspannung korreliert ist.

Die Erfindung wird anhand zweier Figuren näher erläutert.

Beide Fig. zeigen erfindungsgemäße Vorrichtungen.

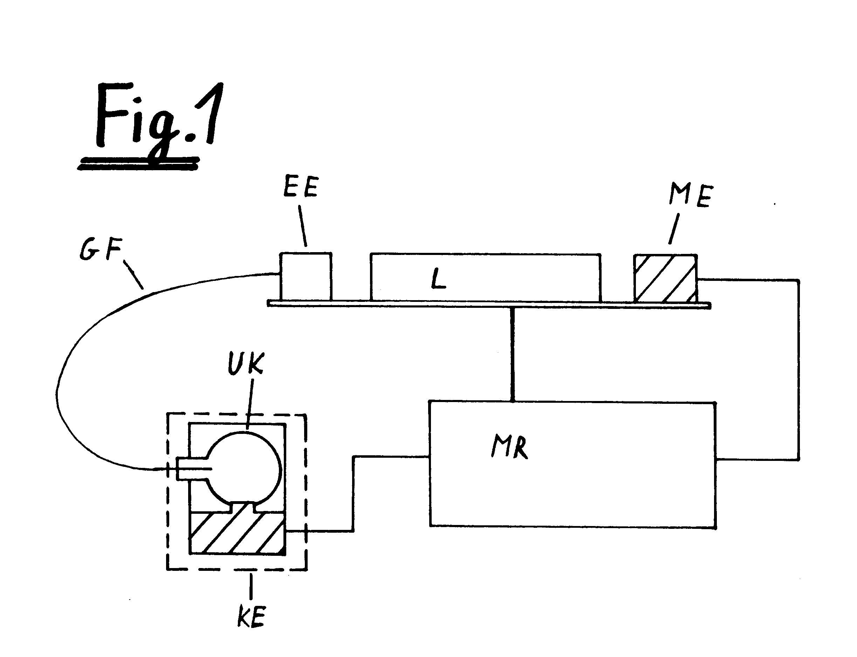

Fig. 1 zeigt eine erfindungsgemäße Vorrichtung zum Regeln der am distalen Ende einer Lichtleitfaser (Glasfaser GF) emittierten Laserenergie. Gezeigt ist der Laserkopf oder der Laser L, die Einkoppeleinheit EE und die Glasfaser GF. Die Regeleinrichtung besteht hier aus der Monitoreinheit ME hinter dem Laser L, der Kalibriereinheit KE, die hier im wesentlichen von der Ulbricht-Kugel UK realisiert ist und der Meß- und Regeleinheit MR, die die Meßergebnisse aus der Monitoreinheit ME, der Kalibriereinheit KE mit der Energie des Lasers L verknüpft und regelt. Das vom Laser L emittierte Licht gelangt über die Einkoppeleinheit EE und die Glasfaser GF an das distale Ende der Glasfaser, das normalerweise an den Behandlungsort geschoben ist. In der Zeichnung befindet sich das distale Ende der Glasfaser GF in der Kalibriereinheit KE.

Durch die nachfolgend beschriebene Kalibrierroutine kann - für die Dauer einer Behandlung - die vom Anwender gewünschte distale Energie innerhalb einer Schwankungsbreite von ± 10 % gewährleistet werden. Vor jeder neuen Behandlung und/oder bei Verwendung eines neuen Fasersystems kann die Kalibrierroutine durchlaufen werden. Die Glasfaser GF wird am Anfang mit Hilfe eines geeigneten Steckers in die Kalibriervorrichtung KE eingeführt, welche sich aus praktischen Gründen am Bedienpult des Geräts befindet.

Als nächstes wird ein Kalibrierprogramm gestartet, bei dem automatisch in z.B. 5 mJ Schritten die ausgekoppelte Laserenergie zwischen 30 und 80 mJ (die Werte können je nach Anwendung des Lasers verschieden sein) eingestellt wird. Während dieser Kalibrierroutine speichert die Meß- und Regeleinheit MR die für die verschiedenen distalen Energien benötigte Lampenspannung sowie die vom Resonator des Laser L mit Hilfe der Monitoreinheit ME gemessene, abgegebene Energie ab. ME und KE werden also gleichzeitig beleuchtet. Auf diese abgespeicherten Werte greift die Meß- und Regeleinheit MR während der Behandlung zurück, um die gewünschte distale Energie bereitzustellen und gegebenenfalls nachzuregeln.

Die Meß- und Regeleinheit MR "weiß" nach der Kalibrierung, welche Energie am distalen Ende der Faser anliegt, wenn eine bestimmte Energie von der zweiten Meßeinheit, der Monitoreinheit ME, im Betrieb registriert wird. So wird erfindungsgemäß im Betrieb eine permanente Echtzeit-Kontrolle der abgegebenen Energie sichergestellt. Sie kann - auch bei Faserschäden - den vorher eingestellten Wert nie überschreiten.

Vorteilhaft ist, daß das erfindungsgemäße System bei der Kalibrierroutine automatisch die richtige Funktion des Fasersystems, der Meßschaltungen sowie der Fasereinkoppelvorrichtung (EE) überprüfen kann, da das Verhältnis von ausgekoppelter Laserenergie zu distaler Energie am Faserende in eine bestimmten Grenze liegen muß, die über die Software in der Meß- und Regeleinheit MR einstellbar ist. Außerdem ist es durch die erfindungsgemäße Vorrichtung möglich, eine reproduzierbare Laserenergie am Faserende sicherzustellen.

Der gesamte Kalibriervorgang kann automatisch gesteuert ablaufen. Der im Kalibriermeßkopf (hier Ulbricht-Kugel UK) gemessene Istwert der Laserenergie am distalen Ende der Glasfaser GF wird mit dem vorgegebenen Pulsenergie-Sollwert verglichen. Bei einer Abweichung wird die Lampenspannung des Lasers L von der Meß- und Regeleinheit MR entsprechend nachgeführt. Die Werte für die Lampenspannung und die Monitorenergie, der Meßwert der Monitoreinheit ME, werden als Sollwert in einer Kalibriertabelle abgespeichert, nachdem die Nachführung abgeschlossen ist. In dieser Tabelle ist jedem einstellbaren Pulsenergiewert jeweils ein Sollwert für die Lampenspannung und für die Monitorenergie zugeordnet. Während der Behandlung dienen die abgespeicherten Werte als Sollvorgaben für die Lampenspannung und die Monitorenergie. Die erstellte Kalibriertabelle wird gespeichert und bleibt damit auch nach Abschluß der Behandlung erhalten. Die Werte können für die nächste Behandlung verwendet werden. Möglich ist ebenso eine Neukalibrierung vor der nächsten Behandlung.

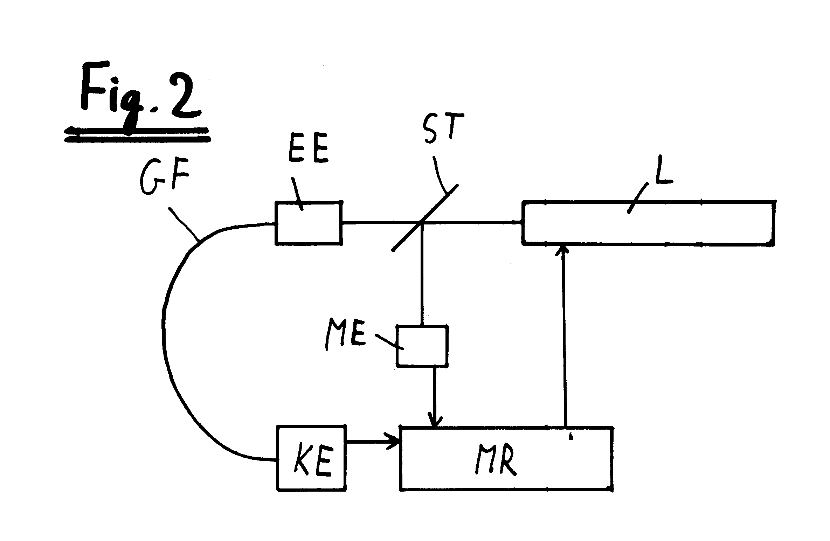

Fig. 2 zeigt eine weitere Ausführungsform der Erfindung, bei der die Monitoreinheit ME nicht hinter dem Laser L angeordnet ist, sondern Licht aus dem Strahlengang erhält. Dazu ist im Strahlengang von der Einkoppeleinheit EE ein Strahlteiler ST angeordnet, der einen Teil des Lichts auf die Monitoreinheit ME lenkt. Der Strahlteiler ST kann z.B. eine schräg gestellte Glasplatte sein, die einen sehr geringen Anteil der Laserstrahlung durch Reflexion auskoppelt.

Außer in der Medizintechnik sind die beanspruchten Vorrichtungen auch bei anderen Laseranwendungen allgemein einsetzbar.

**Patentansprüche**

1. Vorrichtung zum Regeln der am distalen Ende einer Lichtleitfaser (**GF**) emittierten Laserenergie, **gekennzeichnet** durch
   - eine Kalibriereinheit (**KE**) zur Registrierung der distal austretenden Lichtenergie oder Intensität,
   - eine Monitoreinheit (**ME**), die das aus dem Laser (**L**) austretende Licht registriert und
   - eine Meß- und Regeleinheit (**MR**), die während des Kalibrierens eine Relation zwischen der von der Kalibriereinheit (**KE**) registrierten und der von der Monitoreinheit (**ME**) registrierten Laserenergie erstellt und im Betrieb aus der Relation und dem von der Monitoreinheit (**ME**) empfangenen Licht die distal emittierte Energie errechnet und den Laser (**L**) entsprechend in seiner Leistung regelt.

2. Vorrichtung nach Anspruch 1, **dadurch gekennzeichnet,** daß die Kalibriereinheit (**KE**) eine Ulbricht-Kugel (**UK**) enthält, in der eine Photodiode außerhalb des einfallenden Strahls angeordnet ist.

3. Vorrichtung nach Anspruch 1 oder Anspruch 2, **dadurch gekennzeichnet,** daß die Monitoreinheit (**ME**) das aus dem hochreflektierenden Spiegel des Lasers (**L**) austretende Licht registriert.

4. Vorrichtung zum Messen der am distalen Ende einer Lichtleitfaser (**GF**) emittierten Laserenergie, **gekennzeichnet** durch eine Ulbricht-Kugel (**UK**), in der eine Photodiode außerhalb des einfallenden Strahls angeordnet ist.

# Fig.1

EP 0 467 137 A2

# Fig. 2

GF — EE — ST — L

ME

KE → MR